# EUROPÄISCHE PATENTSCHRIFT

(11) **EP 2 371 295 B1**
(45) Veröffentlichungstag und Bekanntmachung des Hinweises auf die Patenterteilung: **08.10.2014**
(21) Anmeldenummer: 11160058.1
(22) Anmeldetag: 28.03.2011
(51) Int. Cl.: A61B 17/06, A61B 17/00, A61B 17/29

(54) **Medizinisches Instrument für einen minimalinvasiven Eingriff**
Medical instrument for a minimally invasive procedure
Instrument médical pour une intervention micro-invasive

(30) Priorität: 01.04.2010 DE 102010013917
(43) Veröffentlichungstag der Anmeldung: 05.10.2011
(73) Patentinhaber: Karl Storz GmbH & Co. KG, 78532 Tuttlingen (DE)
(72) Erfinder: Blocher, Martin, 78532, Tuttlingen (DE); Wagner, Sebastian, 78532, Tuttlingen (DE)

(56) Entgegenhaltungen:
- EP-A1- 2 087 834
- WO-A1-2005/079702

## Beschreibung

Die vorliegende Erfindung bezieht sich auf ein medizinisches Instrument für einen minimalinvasiven Eingriff, insbesondere für einen Eingriff mittels mehrerer durch eine einzige Zugangsöffnung eingeführter Instrumente, und einen Schaft für ein solches medizinisches Instrument.

Minimalinvasive Eingriffe, beispielsweise laparoskopisch chirurgische Eingriffe, wurden ursprünglich über mehrere kleine Öffnungen durchgeführt. Beispielsweise wurden durch eine zentrale Zugangsöffnung ein Endoskop und durch eine, zwei oder mehr seitliche Zugangsöffnungen medizinische Instrumente eingeführt. Die Anordnung eines Endoskops zentral und zweier Instrumente von der Seite wird verschiedentlich auch als Triangulation bezeichnet. Zunehmend wird jedoch versucht, auch die Anzahl der Zugangsöffnungen zu reduzieren. Beispielsweise wird in der laparoskopischen Chirurgie nur noch eine zentrale Zugangsöffnung verwendet, durch die ein Endoskop und in der Regel mehrere Instrumente gleichzeitig eingeführt werden. Aus geometrischen Gründen können diese Instrumente nicht völlig gerade sein. Bei geraden Instrumenten, deren mittlere Abschnitte in einer kleinen Zugangsöffnung angeordnet sind, verhindern oder behindern die vergleichsweise voluminösen Handhabungseinrichtungen an den proximalen Enden ein vollständiges Zusammenführen der distalen Enden. Deshalb wurden Instrumente mit gekrümmtem Schaft entwickelt.

In der WO 2006/100658 A2, in der EP 2 087 834 A1 und in der DE 20 2009 OG'7 592 U1 sind gekrümmte Schäfte von chirurgischen Instrumenten beschrieben. Ebenso in der WO 2005/079702 A1, die ein chirurgisches Instrument mit einem schaft zeigt, der gerade und zusätzlich helikale Abschnitte aufweist. Die zweiteilige Form des Anspruchs 1 basiert auf diesem Dokument.

Gekrümmte Schäfte können gegenüber geraden Schäften eine deutliche Verbesserung darstellen, insbesondere bei minimalinvasiven Eingriffen mit mehreren Instrumenten in einer einzigen Zugangsöffnung. Es bleibt jedoch eine gegenseitige Behinderung der Instrumente, insbesondere der distalen Enden mit den Werkzeugen, der mittleren Abschnitte der Schäfte, die in der Zugangsöffnung angeordnet sind, und der proximalen Enden der Instrumente mit den Handhabungseinrichtungen. Medizinisches Personal muss bei der Handhabung der Instrumente ständig diese potenzielle oder tatsächliche gegenseitige Behinderung berücksichtigen. Dies bedeutet einerseits, dass ein Teil der Aufmerksamkeit des medizinischen Personals ständig auf eine umsichtige und vorausschauende Handhabung der medizinischen Instrumente gerichtet sein muss, und andererseits, dass bestimmte Bewegungen der medizinischen Instrumente aufgrund geometrischer Restriktionen vermieden werden müssen oder nicht ausgeführt werden können. Beides ist einer konzentrierten, effizienten und ermüdungsarmen Arbeit des medizinischen Personals nicht zuträglich.

Eine Aufgabe der vorliegenden Erfindung besteht darin, einen verbesserten Schaft, ein verbessertes medizinisches Instrument und ein verbessertes Operationsbesteck zu schaffen.

Diese Aufgabe wird durch den Gegenstand des unabhängigen Anspruchs gelöst.

Weiterbildungen sind in den abhängigen Ansprüchen angebeben.

Die vorliegende Erfindung beruht auf der Erkenntnis, dass in vielen Situationen eine gegenseitige Behinderung mehrerer medizinischer Instrumente von deren zweidimensionaler Gestalt herrührt. Mit Blick auf die Herstellung, den Transport und die Lagerung medizinischer Instrumente mag es vorteilhaft sein, wenn deren Schäfte zweidimensional geformt sind. Es ist auch nicht offensichtlich, dass eine zweidimensionale Ausgestaltung der Schäfte von medizinischen Instrumenten deren gegenseitige Behinderung fördert bzw. dass eine dreidimensionale Ausgestaltung der Schäfte von medizinischen Instrumenten deren gegenseitige Behinderung reduziert. Eine genaue und unvoreingenommene Analyse von typischen in der Praxis auftretenden Situationen mehrerer medizinischer Instrumente in einer Zugangsöffnung und empirische Untersuchungen mit dreidimensional geformten Schäften ergaben jedoch überraschend eine verminderte gegenseitige Behinderung bereits bei relativ geringen Abweichungen der Schäfte von einer rein zweidimensionalen Formgebung. Abweichungen der Formgebung von einer reinen Zweidimensionalität an einem distalen Abschnitt des Schafts, der für eine Anordnung im Körper vorgesehen ist, an einem mittleren Abschnitt, der für eine Anordnung in einer Zugangsöffnung vorgesehen ist, und einem proximalen Abschnitt, der für eine Anordnung außerhalb des behandelten Körpers vorgesehen ist, haben jeweils spezifische Vorteile.

Ein Schaft für ein medizinisches Instrument für einen minimalinvasiven Eingriff umfasst ein proximales Ende, das mit einer Handhabungseinrichtung mechanisch verbindbar oder verbunden ist, und ein distales Ende, das mit einem Werkzeug verbindbar oder verbunden ist, wobei keine Ebene existiert, von der die Mittelpunkte aller Querschnitte des Schafts einen geringeren Abstand als ein Drittel eines Durchmessers des Schafts haben.

Der Schaft ist starr bzw. bei den Kräften und Momenten, die bei der vorgesehenen Verwendung auftreten, nicht plastisch verformbar. Der Schaft ist insbesondere für einen explorativen, chirurgischen, therapeutischen oder anderen medizinischen Eingriff durch eine einzige Zugangsöffnung ausgebildet.

Die Querschnitte beziehen sich auf Schnittebenen, die von der durch die Mittelpunkte gebildete Mittellinie senkrecht durchstoßen werden. Der Mittelpunkt eines Querschnitts ist unabhängig von etwaigen Hohlräumen im Schaft der geometrische Mittelpunkt der durch die äußere Kontur des Querschnitts begrenzten einfach zusammenhängenden ebenen Fläche. Insbesondere sind alle Querschnitte des Schafts oder zumindest deren äußere Konturen gleich oder im Wesentlichen gleich. Beispielsweise sind alle Querschnitte kreisförmig mit dem gleichen Radius.

Im Fall eines entlang des Schafts variierenden Querschnitts ist hinsichtlich des Vergleichs des Abstands des Mittelpunkts des Querschnitts von einer Ebene mit dem Durchmesser des Schafts der beim betrachteten Querschnitt vorliegende Durchmesser zugrunde zu legen. Bei einem nicht-kreisförmigen Querschnitt wird als Durchmesser der Durchmesser des kleinsten Kreises betrachtet, der den Querschnitt vollständig umschreibt.

Bei dem hier beschriebenen Schaft existiert insbesondere keine Ebene, von der die Mittelpunkte aller Querschnitte des Schafts einen geringeren Abstand als einen halben Durchmesser des Schafts haben.

Bei einigen Ausführungsformen liegen die Mittelpunkte der Querschnitte in einem oder mehreren Abschnitten des Schafts, insbesondere auch in gekrümmten Abschnitten des Schafts, in genau einer Ebene, wobei mindestens ein Abschnitt des Schafts existiert, in dem die Mittelpunkte der Querschnitte einen Abstand von mindestens einem Drittel oder mindestens einer Hälfte des Durchmessers des Schafts haben.

Insbesondere anhand der nachfolgend beschriebenen Ausführungsformen wird deutlich werden, dass die oben beschriebene Abweichung des Schafts von der ebenen Gestalt eine gegenseitige Behinderung der Schäfte zweier medizinischer Instrumente deutlich vermindern oder sogar vermeiden kann. Dabei muss die Abweichung des Schafts von der ebenen Gestalt in vielen Fällen nicht groß sein. Bereits eine Abweichung von einem drittel, einem halben oder einem ganzen Durchmesser des Schafts kann die beschriebenen positiven Wirkungen haben.

Dabei hat sich der durch die Dreidimensionalität bedingte zusätzliche Herstellungsaufwand in einigen Fällen als deutlich niedriger als vorher angenommen herausgestellt. Vor allem bei geringen Abweichungen von einer ebenen Gestalt bleiben auch die Vorteile der ebenen Gestalt hinsichtlich Transport und Lagerung weitgehend erhalten. Allenfalls geringen Nachteilen bzw. einem mäßigen zusätzlichen Aufwand stehen deutliche Vorteile bei der Handhabung gegenüber. Eine gegenseitige Behinderung bzw. Hemmung bzw. Bewegungseinschränkung der Schäfte mehrerer medizinischer Instrumente kann seltener oder mit geringerer Wahrscheinlichkeit auftreten.

Bei einem Schaft, wie er hier beschrieben ist, sind das proximale Ende und das distale Ende parallel zueinander ausgerichtet oder schließen sie einen Winkel von höchstens 10° ein.

Die Richtung eines Endes eines Schafts ist die Richtung des Tangentialvektors der Mittellinie an dem Ende. Eine parallele oder sogar koaxiale Ausrichtung beider Enden eines Schafts kann eine besonders intuitive Handhabung durch medizinisches Personal fördern. Auch bei der oben beschriebenen helikalen Ausbildung eines Bereichs des Schafts können sich an den helikalen Bereich Übergangsbereiche anschließen, so dass beide Enden des Schafts parallel oder sogar koaxial ausgerichtet sein können, selbst wenn der helikale Abschnitt keine ganze Windung umfasst.

Ein Schaft, wie er hier beschrieben ist, weist einen proximalen Bereich, einen mittleren Bereich und einen distalen Bereich auf, wobei der distale Bereich und der proximale Bereich in einer Ebene liegen, von welcher der mittlere Bereich abweicht.

Wie erwähnt ist der mittlere Bereiche des Schafts bei der vorgesehenen Verwendung zusammen mit einem oder mehreren anderen Schäften eines Endoskops und/oder anderer medizinsicher Instrumente in einer engen Zugangsöffnung (beispielsweise einem Trokar bzw. einer Trokarhülse) angeordnet. Wenn zwei Schäfte in diesem mittleren Bereich eine ebene Gestalt aufweisen, können sie linienförmig oder an mehreren voneinander beabstandeten Orten aneinander anliegen. Bei einer Relativbewegung beider Schäfte kann dies den Reibungswiderstand erhöhen oder zu einer diskontinuierlichen Veränderung relativer Drehpunkte und Hebelarme führen. Beides ist in der Regel unerwünscht, da es die Feinfühligkeit und die Präzision, mit der die Schäfte relativ zueinander bewegt werden können, einschränkt.

Wenn die Gestalt von einem oder beiden Schäften im mittleren Bereich von einer Ebene abweicht, können die Schäfte in der Regel nur noch an einem Ort aneinander anliegen. Ein Kippen oder Verschieben beider Schäfte relativ zueinander hat in diesem Fall eine minimale Reibung und keine oder allenfalls eine kontinuierliche Verlagerung einer relativen Drehachse und entsprechend keine oder allenfalls eine kontinuierliche Veränderung der Längen von Hebelarmen zur Folge. Eine Abweichung des Schafts von der ebenen Gestalt kann so die Feinfühligkeit und Präzision der Handhabung und der mittels des Schafts ausgeführten Tätigkeiten erhöhen.

Bei einem Schaft, wie er hier beschrieben, kann der mittlere Bereich helikal ausgebildet sein.

Eine Helix ist eine Raumkurve mit konstanter Krümmung auf einer Mantelfläche eines Kreiszylinders. Ein Bereich des Schafts ist helikal ausgebildet, wenn in dem Bereich die Mittelpunkte aller Querschnitte des Schafts auf einer Helix liegen. Der helikal ausgebildete Bereich des Schafts kann der proximale, der mittlere oder der distale Bereich im Sinne der obigen Beschreibung sein. Ferner kann der gesamte Schaft oder nahezu der gesamte Schaft helikal ausgebildet sein. In Hinblick auf die Herstellung kann es jedoch bei allen hier beschriebenen Ausführungsformen vorteilhaft sein, wenn kurze Abschnitte (mit einer Länge von beispielsweise einigen Millimetern oder wenigen Zentimetern) an den Enden des Schafts gerade sind.

Eine helikale Ausbildung des Schafts oder eines Bereichs des Schafts ermöglicht abhängig vom Radius und der Ganghöhe der Helix einerseits eine Anordnung der distalen Enden mehrerer Schäfte und damit verbundener Werkzeuge ähnlich wie bei der klassischen Triangulation, bei der die Werkzeuge mehrerer medizinischer Instrumente seitlich in das Blickfeld eines zentralen Endoskops reichen. Gleichzeitig liegen zwei Schäfte, die auch im mittleren Bereich mit gleicher Windungsrichtung helikal ausgebildet sind, in der Zugangsöffnung in fast allen relativen Positionen nur an einem Punkt aneinander an. Eine relative Verschiebung oder Verkippung von zwei oder mehr spiralförmigen Schäften in der Zugangsöffnung ist deshalb bei minimaler Reibung und ohne sprunghafte Änderung effektiver Hebellängen möglich.

In der Regel ist eine Verwendung mehrerer Schäfte mit helikal ausgebildeten Bereichen mit gleicher Windungsrichtung, gleichen oder ähnlichen Radien und gleichen oder ähnlichen Ganghöhen vorteilhaft. Besonders positive Erfahrungen liegen mit Schäften vor, die über nahezu ihre gesamte Länge im Wesentlichen eine halbe Windung bilden, wobei das Verhältnis zwischen dem Radius und der Ganghöhe zwischen 1:10 und 1:20 liegt.

Bei einem Schaft, wie er hier beschrieben ist, kann in einem zusammenhängenden Bereich, der mindestens die Hälfte der Länge des Schafts umfasst, die Ableitung des normierten Tangentialvektors der Mittellinie des Schafts entlang der Mittellinie kontinuierlich oder diskontinuierlich in einer Richtung rotieren oder, in geraden Abschnitten, Null betragen.

Wie bereits erwähnt, ist die Mittellinie des Schafts die Menge der Mittelpunkte aller Querschnitte. Ein Beispiel für eine Gestalt des Schafts, bei welcher der normierte Tangentialvektor kontinuierlich in einer Richtung rotiert, ist die erwähnte helikale Gestalt. Abweichend von einer helikalen Gestalt kann die Ableitung des normierten Tangentialvektors entlang der Mittellinie sich sowohl hinsichtlich ihres Betrags als auch hinsichtlich ihrer Richtung sprunghaft bzw. diskontinuierlich ändern. Beispielsweise kann eine Abfolge mehrerer Abschnitte, die jeweils innerhalb einer Ebene gekrümmt sind, unter Umständen einen geringeren Herstellungsaufwand hervorrufen als eine helikale Struktur oder eine andere Struktur, bei welcher der normierte Tangentialvektor kontinuierlich rotiert.

Ein Schaft, wie er hier beschrieben ist, kann einen ersten Abschnitt, in dem der Schaft parallel zu einer ersten Ebene verläuft, und einen zweiten Abschnitt, in dem der Schaft parallel zu einer zweiten Ebene verläuft, umfassen, wobei die erste Ebene und die zweite Ebene nicht parallel zueinander sind.

Insbesondere ist der Schaft sowohl im ersten Abschnitt als auch im zweiten Abschnitt gekrümmt. Mathematisch formuliert hat das Vektorprodukt aus dem Tangentialvektor und der Ableitung des Tangentialvektors im ersten Abschnitt eine erste Richtung und im zweiten Abschnitt eine zweite Richtung, die von der ersten Richtung verschieden ist. Eine Krümmung innerhalb einer Ebene kann in manchen Fällen mit geringerem Herstellungsaufwand erzeugt werden, so dass eine Abfolge von Abschnitten, innerhalb derer der Schaft jeweils parallel zu einer Ebene verläuft, kostengünstig erzeugt werden kann.

Die erste Ebene und die zweite Ebene schließen insbesondere einen Winkel von mindestens 10° ein.

Der Winkel zwischen zwei Ebenen ist der Winkel zwischen ihren Flächennormalen. Größere Winkel von mindestens 20° oder mindestens 30° oder mindestens 60° können vorteilhaft sein.

Ein medizinisches Instrument umfasst einen Schaft, wie er hier beschrieben ist, und zumindest entweder eine Handhabungseinrichtung, die mit dem proximalen Ende des Schafts verbindbar oder verbunden ist, oder ein Werkzeug, das mit dem distalen Ende des Schafts verbindbar oder verbunden ist.

Ein Operationsbesteck umfasst zwei medizinische Instrumente, wie sie hier beschrieben sind, wobei bei den Schäften beider medizinischer Instrumente in einander hinsichtlich der Abstände von den distalen Enden der Schäfte entsprechenden Bereichen die Ableitungen der normierten Tangentialvektoren der Mittellinien der Schäfte entlang der Mittellinie kontinuierlich oder diskontinuierlich in der gleichen Richtung rotieren.

Die gleichsinnige Rotation der Ableitung der normierten Tangentialvektoren reduziert die gegenseitige Behinderung zweier Schäfte.

Bei einem Operationsbesteck, wie es hier beschrieben ist, sind die Schäfte beider medizinsicher Instrumente in einander hinsichtlich der Abstände von den distalen Enden der Schäfte entsprechenden Bereichen helikal ausgebildet und weisen die gleiche Windungsrichtung auf.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine weitere schematische Darstellung des medizinischen Instruments aus Figur 1;
- Figur 3: eine weitere schematische Darstellung des medizinischen Instruments aus den Figuren 1 und 2;
- Figur 4: eine schematische Darstellung eines weiteren medizinischen Instruments;
- Figur 5: eine weitere schematische Darstellung des medizinischen Instruments aus Figur 4;
- Figur 6: eine weitere schematische Darstellung des medizinischen Instruments aus den Figuren 4 und 5;
- Figur 7: eine schematische Darstellung eines weiteren medizinischen Instruments;
- Figur 8: eine weitere schematische Darstellung des medizinischen Instruments aus Figur 7;
- Figur 9: eine weitere schematische Darstellung des medizinischen Instruments aus den Figuren 7 und 8;
- Figur 10: eine schematische Darstellung eines weiteren medizinischen Instruments;
- Figur 11: eine weitere schematische Darstellung des medizinischen Instruments aus Figur 10;
- Figur 12: eine weitere schematische Darstellung des medizinischen Instruments aus den Figuren 10 und 11;
- Figur 13: eine schematische Darstellung zweier medizinischer Instrumente bei der vorgesehenen Verwendung.

### Beschreibung der Ausführungsformen

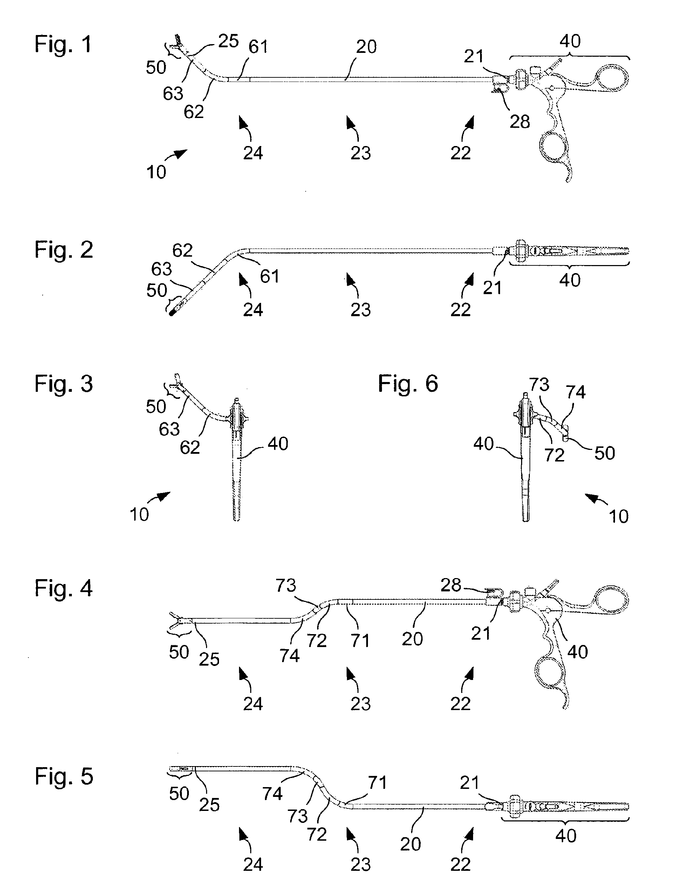

Die Figuren 1 bis 12 zeigen schematische Darstellungen von vier verschiedenen medizinischen Instrumenten. Jeweils drei aufeinanderfolgende Figuren zeigen schematische axonometrische Darstellungen eines medizinischen Instruments aus drei verschiedenen Perspektiven bzw. Richtungen. Die Zeichenebenen der drei sich jeweils auf das gleiche medizinische Instrument beziehenden Figuren 1 bis 3 bzw. 4 bis 6 bzw. 7 bis 9 bzw. 10 bis 12 sind jeweils senkrecht zueinander. Die Zeichenebenen der Figuren 1, 4, 7 und 10 entsprechen einander. Die Zeichenebenen der Figuren 2, 5, 8 und 11 entsprechen einander. Die Zeichenebenen der Figuren 3, 6, 9 und 12 entsprechen einander.

Die Instrumente der Figuren 1-3 sowie 7-9 sind nicht erfindungsgemäße Ausführungsformen.

Jedes der in den Figuren 1 bis 3 bzw. 4 bis 6 bzw. 7 bis 9 bzw. 10 bis 12 dargestellten medizinischen Instrumente 10 umfasst einen Schaft 20 mit einem proximalen Ende 21 und einem distalen Ende 25 und einem im Wesentlichen konstanten kreisförmigen Querschnitt zwischen dem proximalen Ende 21 und dem distalen Ende 25. Am proximalen Ende 21 kann der Schaft 20 jeweils einen Spülanschluss 28 aufweisen.

Mit dem proximalen Ende 21 ist eine Handhabungseinrichtung 40 verbunden, mit dem distalen Ende 25 des Schafts 20 ist ein Werkzeug 50 verbunden. Sowohl die Handhabungseinrichtung 40 als auch das Werkzeug 50 können jeweils dauerhaft bzw. nicht zerstörungsfrei lösbar mit dem Schaft 20 verbunden sein. Sowohl die Handhabungseinrichtung 40 als auch das Werkzeug 50 können jeweils alternativ zerstörungsfrei lösbar mit dem Schaft 20 verbunden sein, beispielsweise über Bajonett- und/oder Rastverbindungen.

Bei den dargestellten medizinischen Instrumenten sind jeweils eine Steckverbindung zwischen dem proximalen Ende 21 des Schafts 20 und der Handhabungseinrichtung 40 und eine Rotierbarkeit des Schafts in der Handhabungseinrichtung 40 vorgesehen, wobei eine lösbare Rastverbindung den Schaft 20 an der Handhabungseinrichtung 40 hält. Die Handhabungseinrichtungen 40 aller dargestellter medizinischer Instrumente 10 weisen einander entsprechende Merkmale auf, auf die nachfolgend nicht näher eingegangen wird.

Teilweise wird nachfolgend zwischen einem proximalen Bereich 22, einem mittleren Bereich 23 und einem distalen Bereich 24 des Schafts 20 unterschieden. Der proximale Bereich 22 erstreckt sich bis zum oder im Wesentlichen bis zum proximalen Ende 21 des Schafts 20. Der distale Bereich 24 erstreckt sich bis zum oder im Wesentlichen bis zum distalen Ende 25. Der mittlere Bereich 23 erstreckt sich im Wesentlichen zwischen dem proximalen Bereich 22 und dem distalen Bereich 24. Bei der vorgesehenen Verwendung der medizinischen Instrumente 10 ist der proximale Bereich 22 des Schafts 20 dafür vorgesehen, außerhalb eines zu behandelnden Körpers angeordnet zu sein; der distale Bereich 24 ist dafür vorgesehen, in einem natürlichen oder künstlichen Hohlraum in dem zu behandelnden Körper angeordnet zu sein; der mittlere Bereich 23 ist dafür vorgesehen, in einer Zugangsöffnung angeordnet zu sein, beispielsweise in einem Trokar bzw. einer Trokarhülse.

Die Figuren 1 bis 3 zeigen schematische axonometrische Darstellungen eines medizinischen Instruments 10, dessen Schaft 20 im proximalen Bereich 22 und im mittleren Bereich 23 gerade ist. An den geraden Bereich schließen sich distal zwei gekrümmte Abschnitte 61, 62 und ein weiterer, kurzer gerader Abschnitt 63 an. Innerhalb des ersten gekrümmten Abschnitts 61 und innerhalb des zweiten gekrümmten Abschnitts 62 weist der Schaft jeweils eine im Wesentlichen ebene Gestalt auf. Innerhalb des ersten gekrümmten Abschnitts 61 liegen die Mittelpunkte aller Querschnitte des Schafts 20 in einer einzigen, ersten Ebene, innerhalb des zweiten gekrümmten Abschnitts 62 liegen die Mittelpunkte aller Querschnitte des Schafts 20 in einer einzigen, zweiten Ebene. Aufgrund der Krümmung des ersten gekrümmten Abschnitts 61 und des zweiten gekrümmten Abschnitts 62 sind sowohl die erste Ebene als auch die zweite Ebene eindeutig definiert. Aufgrund der geraden Gestalt des Schafts 20 proximal des ersten gekrümmten Abschnitts 61 und distal des zweiten gekrümmten Abschnitts 62 liegen die Mittelpunkte aller Querschnitte des ersten gekrümmten Abschnitts 61 und proximal desselben in der ersten Ebene und die Mittelpunkte aller Querschnitte des Schafts 20 im zweiten gekrümmten Abschnitt 62 und distal desselben in der zweiten Ebene.

Die erste Ebene und die zweite Ebene sind nicht parallel zueinander. In Figur 1 ist erkennbar, dass die erste Ebene senkrecht zur Zeichenebene der Figur 1 ist, weshalb der erste gekrümmte Abschnitt 61 in Figur 1 gerade erscheint. In Figur 2 ist erkennbar, dass die zweite Ebene senkrecht zur Zeichenebene der Figur 2 ist, weshalb der zweite gekrümmte Abschnitt 62 in Figur 2 gerade erscheint.

In der Zusammenschau der Figuren 1 bis 3 ist erkennbar, dass keine Ebene existiert, in der die Mittelpunkte aller Querschnitte des Schafts 20 liegen. Der distale Bereich 24 des Schafts 20 weist vielmehr eine angedeutete Schraubenform auf. Die Ableitung des normierten Tangentialvektors der aus den Mittelpunkten aller Querschnitte gebildeten Mittellinie des Schafts 20 liegt im ersten gekrümmten Abschnitt 61 in der Zeichenebene der Figur 2 und ist am proximalen Ende des zweiten gekrümmten Abschnitts 62 senkrecht zur Zeichenebene der Figur 2. Die Ableitung des Tangentialvektors der Mittellinie rotiert am Übergang zwischen dem ersten gekrümmten Abschnitt 61 und dem zweiten gekrümmten Abschnitt 62 sprungartig um 90° im Uhrzeigersinn.

Die Gestalt des Schafts 20 des medizinischen Instruments 10 aus den Figuren 1 bis 3 ermöglicht ein kollisionsfreies bzw. behinderungsfreies Kreuzen des distalen Bereichs 24 des Schafts 20 mit dem distalen Bereich eines Schafts eines weiteren medizinischen Instruments, insbesondere wenn der Schaft des weiteren medizinischen Instruments gerade ist oder zumindest im distalen Bereich schraubenförmig oder näherungsweise schraubenförmig mit der gleichen Windungsrichtung ausgebildet ist. Besonders vorteilhaft kann eine gleichzeitige Verwendung zweier gleicher medizinischer Instrumente 10 sein, die beide der obigen Darstellungen anhand der Figuren 1 bis 3 entsprechen.

Die Figuren 4 bis 6 zeigen schematische axonometrische Darstellungen eines weiteren medizinischen Instruments 10, das in einigen Merkmalen dem oben anhand der Figuren 1 bis 3 dargestellten medizinischen Instrument ähnelt. Abweichend von dem oben anhand der Figuren 1 bis 3 dargestellten medizinischen Instrument ist der Schaft 20 des in den Figuren 4 bis 6 dargestellten medizinischen Instruments 10 im proximalen Bereich 22 und im distalen Bereich 24 gerade und im mittleren Bereich 23 gekrümmt.

Im mittleren Bereich 23 weist der Schaft 20 vier gekrümmte Abschnitte 71, 72, 73, 74 auf, innerhalb derer die Mittelpunkte aller Querschnitte des Schafts 20 jeweils in einer Ebene liegen. Eine erste Ebene, in der die Mittelpunkte aller Querschnitte des Schafts 20 innerhalb des ersten gekrümmten Abschnitts 71 und proximal desselben liegen, ist senkrecht zur Zeichenebene der Figur 4. Deshalb erscheint der erste gekrümmte Abschnitt 71 in Figur 4 gerade. In Figur 6 ist erkennbar, dass eine zweite Ebene, in der die Mittelpunkte aller Querschnitte des Schafts 20 im zweiten gekrümmten Abschnitt 72 liegen, und eine vierte Ebene, in der die Mittelpunkte aller Querschnitte des Schafts 20 im vierten gekrümmten Abschnitt 74 liegen, jeweils senkrecht zur Zeichenebene der Figur 6 sind. Deshalb erscheinen sowohl der zweite gekrümmte Abschnitt 72 als auch der vierte gekrümmte Abschnitt 74 in Figur 6 jeweils gerade.

Bei genauer Betrachtung der Figuren 4 bis 6 ist erkennbar, dass der Schaft 20 im mittleren Bereich 23 näherungsweise eine rechtsdrehende Schraubenform bzw. helikale Form aufweist. Die Ableitung des normierten Tangentialvektors der aus den Mittelpunkten aller Querschnitte gebildeten Mittellinie des Schafts 20 rotiert entlang dieser Mittellinie im Uhrzeigersinn diskontinuierlich. An den Übergängen zwischen den gekrümmten Abschnitten 71, 72, 73, 74 rotiert die Richtung der Ableitung des normierten Tangentialvektors jeweils sprunghaft um Winkel zwischen 20° und 120° nach rechts bzw. im Uhrzeigersinn.

Die Gestalt des Schafts 20 des anhand der Figuren 4 bis 6 dargestellten medizinischen Instruments 10 erleichtert eine reibungsarme und ungehemmte Relativbewegung des Schafts 20 des dargestellten medizinischen Instruments 10 und des oder der Schäfte von einem oder mehreren weiteren medizinischen Instrumenten, die gemeinsam in einer engen Zugangsöffnung angeordnet sind. Dies gilt insbesondere, wenn der oder die Schäfte des oder der weiteren medizinischen Instrumente im mittleren Bereich gerade oder ähnlich gekrümmt sind wie der Schaft 20 des anhand der Figuren 4 bis 6 dargestellten medizinischen Instruments. Vorteilhaft kann insbesondere eine schraubenförmige bzw. helikale oder näherungsweise schraubenförmige bzw. helikale Ausbildung mit gleicher Windungsrichtung alle Schäfte im mittleren Bereich sein. Diese Gestalt ermöglicht bei vielen oder sogar den meisten relativen Anordnungen mehrerer Schäfte eine gegenseitige Berührung in jeweils nur einem Punkt bzw. an jeweils nur einem Ort. Dies hat einerseits im Vergleich zu einer möglichen gegenseitigen Berührung zweier Schäfte in jeweils mindestens zwei voneinander beabstandeten Punkten eine deutlich verminderte Reibung zur Folge. Ein weiterer Vorteil kann darin bestehen, dass, solange eine gegenseitige Berührung zweier Schäfte nur in einem Punkt vorliegt, eine plötzliche Verlagerung einer momentanen Achse einer relativen Rotation zweier Schäfte und eine plötzliche Veränderung der Längen von Hebelarmen nicht möglich ist.

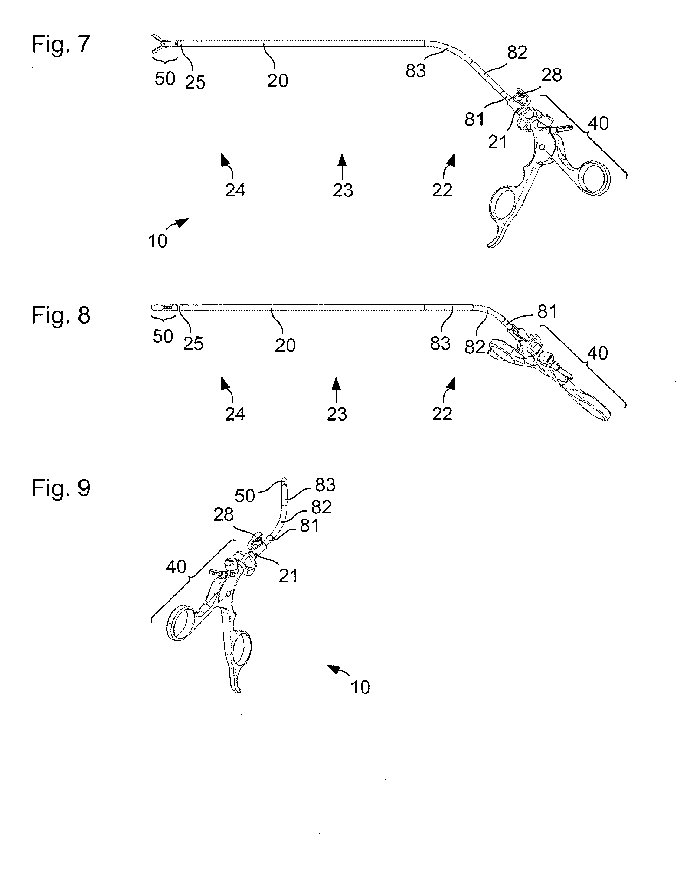

Die Figuren 7 bis 9 zeigen schematische axonometrische Darstellungen eines medizinischen Instruments 10, das in einigen Merkmalen den oben anhand der Figuren 1 bis 3 bzw. 4 bis 6 dargestellten medizinischen Instrumenten ähnelt. Im Unterschied zu den oben anhand der Figuren 1 bis 3 und 4 bis 6 dargestellten medizinischen Instrumenten ist der Schaft 20 des in den Figuren 7 bis 9 dargestellten medizinischen Instruments 10 im proximalen Bereich 22 gekrümmt und im mittleren Bereich 23 und im distalen Bereich 24 gerade.

Im proximalen Bereich 22 weist der Schaft 20 einen kurzen geraden Abschnitt 81, einen ersten gekrümmten Abschnitt 82 und einen zweiten gekrümmten Abschnitt 83 auf. Im ersten gekrümmten Abschnitt 82 und im proximal anschließenden kurzen geraden Abschnitt 81 des Schafts 20 liegen die Mittelpunkte aller Querschnitte des Schafts 20 in einer einzigen, ersten Ebene. Im zweiten gekrümmten Abschnitt 83 und distal desselben liegen die Mittelpunkte aller Querschnitte des Schafts 20 in einer zweiten Ebene. Im Vergleich der Figuren 7 und 8 ist erkennbar, dass die erste Ebene senkrecht zur Zeichenebene der Figur 7 ist. Deshalb erscheint der erste gekrümmte Abschnitt 82 in Figur 7 gerade. Ferner ist erkennbar, dass die zweite Ebene senkrecht zur Zeichenebene der Figur 8 ist. Deshalb erscheint der zweite gekrümmte Abschnitt 83 in Figur 8 gerade.

Im Vergleich der Figuren 7 bis 9 ist erkennbar, dass der Schaft 20 des in den Figuren 7 bis 9 dargestellten medizinischen Instruments 10 im proximalen Bereich 22 näherungsweise die Form einer linksgängigen Schraube bzw. Helix aufweist. Die Ableitung des normierten Tangentialvektors der durch die Mittelpunkte aller Querschnitte der gebildeten Mittellinie des Schafts 20 rotiert am Übergang zwischen dem ersten gekrümmten Abschnitt 82 und dem zweiten gekrümmten Abschnitt 83 sprungartig um ca. 90° gegen den Uhrzeigersinn.

Die Gestalt des Schafts 20 des anhand der Figuren 7 bis 9 dargestellten medizinischen Instruments 10 kann eine besonders behinderungsarme oder behinderungsfreie Relativbewegung der Schäfte mehrerer medizinischer Instrumente, die gleichzeitig in einer Zugangsöffnung verwendet werden, ermöglichen. Dies gilt insbesondere, wenn das anhand der Figuren 7 bis 9 dargestellte medizinische Instrument 10 zusammen mit einem oder mehreren weiteren medizinischen Instrumenten verwendet wird, deren Schäfte im proximalen Bereich entsprechend oder ähnlich dem Schaft 20 des anhand der Figuren 7 bis 9 dargestellten medizinischen Instruments 10 oder gerade ausgebildet sind. Insbesondere kann die dargestellte Gestalt des Schafts 20 ein Kreuzen der proximalen Bereiche zweier Schäfte ohne seitlichen Versatz ermöglichen oder erleichtern.

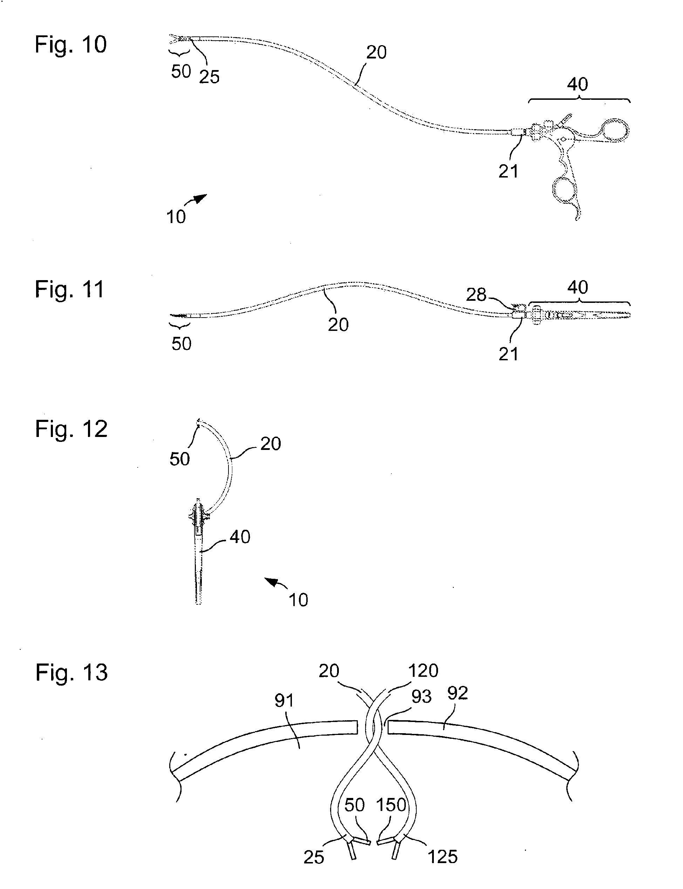

Die Figuren 10 bis 12 zeigen schematische axonometrische Darstellungen eines weiteren medizinischen Instruments 10, das in einigen Merkmalen den oben anhand der Figuren 1 bis 3 bzw. 4 bis 6 bzw. 7 bis 9 dargestellten medizinischen Instrumenten ähnlich ist. Abweichend von den oben anhand der Figuren 1 bis 9 dargestellten medizinischen Instrumenten, weist der Schaft 20 des in den Figuren 10 bis 12 dargestellten medizinischen Instruments in einem großen, mittleren Bereich seiner Länge eine im Wesentlichen helikale Gestalt auf. Übergangsbögen und gerade Abschnitte am proximalen Ende 21 und am distalen Ende 25, die in den Figuren kaum erkennbar sind, bewirken, dass das proximale Ende 21 und das distale Ende 25 des Schafts 20 die gleiche Richtung haben bzw. parallel zueinander sind.

Vorteile des in den Figuren 10 bis 12 dargestellten medizinischen Instruments 10 ähneln oder entsprechen weitgehend Vorteilen der oben anhand der Figuren 1 bis 9 dargestellten medizinischen Instrumenten oder deren Kombination. Insbesondere können die Schäfte zweier oder dreier medizinischer Instrumente, wie sie anhand der Figuren 10 bis 12 oder anhand der Figuren 4 bis 6 dargestellt sind, in einer Zugangsöffnung so angeordnet werden, dass sie sich jeweils gegenseitig nur in einem Punkt bzw. an einem Ort berühren. Dies ist beispielsweise der Fall, wenn zwei Schäfte um ca. 180° gegeneinander verdreht oder drei Schäfte um jeweils ca. 120° gegeneinander verdreht in der Zugangsöffnung angeordnet werden. Aufgrund der dargestellten Gestaltung der Schäfte kreuzen sich diese in der Zugangsöffnung unter großen Winkeln, so dass auch bei größeren relativen Verdrehungen oder Verschiebungen eine gegenseitige Berührung zweier Schäfte immer nur in einem Punkt bzw. an einem Ort vorliegt.

Gleichzeitig kann die dargestellte nicht-ebene Formgebung des Schafts von mindestens einem der gleichzeitig verwendeten medizinischen Instrumente ein Kreuzen der distalen Bereiche 24 und/oder der proximalen Bereiche 22 der Schäfte 20 ohne einen relativen seitlichen Versatz ermöglichen.

Figur 13 zeigt eine schematische Darstellung eines Beispiels einer gleichzeitigen Verwendung zweier medizinischer Instrumente, wie sie beispielsweise oben anhand der Figuren 4 bis 6 oder 10 bis 12 dargestellt sind. Die medizinischen Instrumente werden beispielsweise für einen explorativen oder chirurgischen minimalinvasiven Eingriff in einem künstlichen oder natürlichen Hohlraum 91 im Körper eines Patienten verwendet. Der Hohlraum 91 wird von einer Wand 92 begrenzt, in der eine natürliche oder künstliche Zugangsöffnung 93 vorliegt. Die Wand 92 ist beispielsweise die Bauchdecke des Patienten.

Schäfte 20, 120 zweier medizinischer Instrumente sind durch die Zugangsöffnung 93 in den Hohlraum 91 eingeführt. Im mittleren Bereich bzw. im Bereich der Zugangsöffnung 93 sind die beiden Schäfte 20, 120 in der Art einer Doppelhelix umeinander gewunden angeordnet. Bei ausreichenden Krümmungsradien der Schäfte 20, 120 berühren die Schäfte 20, 120 einander in höchstens einem Punkt.

Wenn beide Schäfte 20, 120 eine ebene Gestalt aufwiesen, d. h. die Mittelpunkte aller Querschnitte des Schafts 20 in einer ersten Ebene und die Mittelpunkte aller Querschnitte des Schafts 120 in einer zweiten Ebene lägen, schnitten sich diese beiden Ebenen im Bereich der Zugangsöffnung 93, könnten aber nicht parallel zueinander sein. Die distalen Enden 25, 125 der Schäfte 20, 120 und die daran angeordneten Werkzeuge 50, 150 könnten deshalb nicht zusammengeführt werden. Bei den meisten minimalinvasiven Eingriffen ist es jedoch erforderlich, dass die Werkzeuge 50, 150 an den distalen Enden 25, 125 der Schäfte 20, 120 von zwei oder mehr medizinischen Instrumenten zumindest bis auf einen sehr kleinen gegenseitigen Abstand aneinander angenähert werden können.

Im Fall ebener Schäfte 20, 120 wäre abweichend von der Darstellung in Figur 13 eine parallele Anordnung der Ebenen der Schäfte 20, 120 denkbar. Dann können die Schäfte 20, 120 einander jedoch in zwei Punkten berühren, was die oben beschriebenen Nachteile hat.

Erst die Abweichung von mindestens einem der Schäfte 20, 120 von einer ebenen Gestalt ermöglicht gleichzeitig die in Figur 13 dargestellte einander zumindest teilweise umschlingende Anordnung der beiden Schäfte 20, 120 in der Zugangsöffnung 93 und eine beliebige Annäherung der distalen Enden 25, 125 der Schäfte 20, 120 und der daran angeordneten Werkzeuge.

### Bezugszeichen

- 10 Medizinisches: Instrument

- 20: Schaft des medizinischen Instruments 10
- 21: proximales Ende des Schafts 20 des medizinischen Instruments 10
- 22: proximaler Bereich des Schafts 20 des medizinischen Instruments 10
- 23: mittlerer Bereich des Schafts 20 des medizinischen Instruments 10
- 24: distaler Bereich des Schafts 20 des medizinischen Instruments 10
- 25: distales Ende des Schafts 20 des medizinischen Instruments 10
- 28: Spülanschluss am Schaft 20

- 40: Handhabungseinrichtung des medizinischen Instruments 10

- 50: Werkzeug des medizinischen Instruments 10

- 61: erster gekrümmter Abschnitt im distalen Bereich 24 des Schafts 20
- 62: zweiter gekrümmter Abschnitt im distalen Bereich 24 des Schafts 20
- 63: gerader Abschnitt im distalen Bereich 24 des Schafts 20

- 71: erster gekrümmter Abschnitt im mittleren Bereich 23 des Schafts 20
- 72: zweiter gekrümmter Abschnitt im mittleren Bereich 23 des Schafts 20
- 73: dritter gekrümmter Abschnitt im mittleren Bereich 23 des Schafts 20
- 74: vierter gekrümmter Abschnitt im mittleren Bereich 23 des Schafts 20

- 81: gerader Abschnitt im proximalen Bereich 22 des Schafts 20
- 82: erster gekrümmter Abschnitt im proximalen Bereich 22 des Schafts 20
- 83: zweiter gekrümmter Abschnitt im proximalen Bereich 22 des Schafts 20

- 91: Hohlraum
- 92: Wand
- 93: Zugangsöffnung

- 120: Schaft eines weiteren medizinischen Instruments
- 125: distales Ende des Schafts 120 des weiteren medizinischen Instruments
- 150: Werkzeug am distalen Ende des Schafts 120

## Patentansprüche

1. **Schaft** (20) für ein medizinisches Instrument (10) für einen minimalinvasiven Eingriff, unit:
einem **proximalen Ende** (21), das mit einer Handhabungseinrichtung (40) mechanisch verbindbar oder verbunden ist;
einem **distalen Ende** (25), das mit einem Werkzeug (50) verbindbar oder verbunden ist,
wobei keine Ebene existiert, von der die Mittelpunkte aller Querschnitte des Schafts (20) einen geringeren Abstand als ein **Drittel** eines Durchmessers des Schafts (20) haben,
wöbei der Schaft (20) einen proximalen Bereich (22), einen mittleren Bereich (23) und einen distalen Bereich (24) aufweist, **dadurch gekennzeichnet, daß**
das proximale **Ende** (21) und das distale **Ende** (25) des Schafts (20) **parallel** zu einander ausgerichtet sind oder einen Winkel von höchstens 10 Grad zu einander einnehmen, und daß der distale Bereich (24) und der proximale Bereich (22) in einer Ebene liegen, von welcher der **mittlere Bereich** (23) abweicht.

2. Schaft (20) nach dem vorangehenden Anspruch, bei dem keine Ebene existiert, von der die Mittelpunkte aller Querschnitte des Schaft (20) einen geringeren Abstand als einen **halben Durchmesser** des Schafts (20) haben.

3. Schaft (20) nach einem der vorangehenden Ansprüche, bei dem der mittlere Bereich des Schafts (20) **helikal** ausgebildet ist.

4. Schaft (20) nach einem der vorangehenden Ansprüche, bei dem in einem zusammenhängenden Bereich, der mindestens die Hälfte der Länge des Schafts (20) umfasst, die Ableitung des normierten Tangcntialvektors der Mittellinie des Schafts (20) entlang der Mittellinie kontinuierlich oder diskontinuierlich in einer Richtung rotiert oder Null beträgt.

5. Schaft (20) nach einem der vorangehenden Ansprüche, wobei der Schaft (20) einen ersten Abschnitt (61; 71; 82), in dem der Schaft (20) parallel zu einer **ersten Ebene** verläuft, und einen zweiten Abschnitt (62; 72; 83), in dem der Schaft (20) parallel zu einer **zweiten Ebene** verläuft, umfasst, wobei die erste Ebene und die zweite Ebene **nicht parallel** zu einander sind.

6. Schaft (20) nach dem vorangehenden Anspruch, bei dem die erste Ebene und die zweite Ebene einen Winkel von mindestens **10 Grad** cinschließcn.

## Claims

1. Shaft (20) for a medical instrument (10) for a minimally invasive procedure, with:
a proximal end (21), which is mechanically connectable or connected to a manipulation device (40),
a distal end (25), which is connectable or connected to a tool (50),
wherein no plane exists from which the centre points of all cross sections of the shaft (20) are at a distance less than one third of a diameter of the shaft (20),
wherein the shaft (20) has a proximal area (22), a central area (23) and a distal area (24),
**characterized in that** the proximal end (21) and the distal end (25) of the shaft (20) are oriented parallel to each other or are at an angle of at most 10 degrees to each other,
and **in that** the distal area (24) and the proximal area (22) lie in a plane from which the central area (23) deviates.

2. Shaft (20) according to the preceding claim, in which no plane exists from which the centre points of all cross sections of the shaft (20) are at a distance less than a half diameter of the shaft (20).

3. Shaft (20) according to one of the preceding claims, in which the central area of the shaft (20) is of helical configuration.

4. Shaft (20) according to one of the preceding claims, in which, in a continuous area that includes at least half of the length of the shaft (20), the derivative of the normed tangential vector of the midline of the shaft (20) rotates continuously or discontinuously along the midline in one direction or is equal to zero.

5. Shaft (20) according to one of the preceding claims, wherein the shaft (20) comprises a first segment (61; 71; 82), in which the shaft (20) runs parallel to a first plane, and a second segment (62; 72; 83), in which the shaft (20) runs parallel to a second plane, wherein the first plane and the second plane are not parallel to each other.

6. Shaft (20) according to the preceding claim, in which the first plane and the second plane form an angle of at least 10 degrees.

## Revendications

1. Tige (20) pour un instrument médical (10) pour une chirurgie mini-invasive, comprenant :
une extrémité proximale (21) qui peut être connectée ou qui est connectée mécaniquement à un dispositif de manipulation (40) ;
une extrémité distale (25) qui peut être connectée ou qui est connectée à un outil (50),
aucun plan n'existant depuis lequel les centres de toutes les sections transversales de la tige (20) sont espacés d'une distance inférieure à un tiers d'un diamètre de la tige (20),
la tige (20) présentant une région proximale (22), une région centrale (23) et une région distale (24), **caractérisée en ce que**
l'extrémité proximale (21) et l'extrémité distale (25) de la tige (20) sont orientées parallèlement l'une à l'autre ou sont orientées suivant un angle de 10 degrés maximum l'une par rapport à l'autre, et **en ce que** la région distale (24) et la région proximale (22) se situent dans un plan duquel s'écarte la région centrale (23).

2. Tige (20) selon la revendication précédente, dans laquelle aucun plan n'existe depuis lequel les centres de toutes les sections transversales de la tige (20) sont espacés d'une distance inférieure à un demi-diamètre de la tige (20).

3. Tige (20) selon l'une quelconque des revendications précédentes, dans laquelle la région centrale de la tige (20) est réalisée sous forme hélicoïdale.

4. Tige (20) selon l'une quelconque des revendications précédentes, dans laquelle, dans une région continue, qui comprend au moins la moitié de la longueur de la tige (20), la dérivée du vecteur tangentiel normé de l'axe médian de la tige (20) le long de l'axe médian tourne de manière continue ou discontinue dans un sens ou est nulle.

5. Tige (20) selon l'une quelconque des revendications précédentes, dans laquelle la tige (20) comprend une première portion (61 ; 71 ; 82) dans laquelle la tige (20) s'étend parallèlement à un premier plan et une deuxième portion (62 ; 72 ; 83) dans laquelle la tige (20) s'étend parallèlement à un deuxième plan, le premier plan et le deuxième plan n'étant pas parallèles l'un à l'autre.

6. Tige (20) selon la revendication précédente, dans laquelle le premier plan et le deuxième plan forment un angle d'au moins 10 degrés.
